# EUROPEAN PATENT APPLICATION

(11) **EP 4 666 963 A1**
(43) Date of publication of application: **24.12.2025**
(21) Application number: 24755943.8
(22) Date of filing: 26.01.2024
(51) Int. Cl.: A61B 17/12

(54) **PULLING CONTROL APPARATUS FOR VASCULAR CLOSURE DEVICE**

(30) Priority: 14.02.2023 CN 202310108948
(71) Applicant: Shanghai Kegang Medical Technology Co., Ltd, Shanghai Songjiang District 201601 (CN)
(72) Inventor: RU, Chengtao, Shanghai 201601 (CN); MA, Changsheng, Shanghai 201601 (CN); DONG, Jianzeng, Shanghai 201601 (CN)
(74) Representative: Cameron Intellectual Property Ltd
(86) International application number: PCT/CN2024/074261
(87) International publication number: WO 2024/169568

(57) **Abstract**

A pulling control apparatus for a vascular closure device, comprising: a housing, a spring [33] disposed within the housing for providing tensile force and tensile displacement, a rotating component [34] and pressing component [35] assembly for controlling different states, wherein the rotating component [34] and pressing component [35] are axially limited after connection and can rotate freely in the circumferential direction; a pulling wire [11] can pass through the interior of the device and is fixed to a fixed component [36] at the rear end of the pressing component [35]; by pressing the pressing component, the rotating component [34] switches positions, relying on the spring [33] to provide tensile force and transmitting the tensile force to the front end through the pulling wire **[11].** The present apparatus is safer and easier to use while avoiding device failure.

## Description

### FIELD OF THE INVENTION

The present application relates to the field of medical devices, particularly to a pulling control apparatus for a vascular closure device.

### BACKGROUND

Interventional procedures have become increasingly common and are often preferred by physicians and patients due to their minimal trauma, operational convenience, and reduced patient risk. Common procedures such as coronary and peripheral angiography, atrial fibrillation ablation, angioplasty, cerebral angiography, and cardiac valve repair (replacement) are all performed using interventional techniques. Interventional procedures involve the introduction of specialized catheters, guidewires, and other precision instruments into the human body under the guidance of medical imaging equipment for the diagnosis and localized treatment of internal pathologies. Therefore, interventional procedures generally require vascular puncture of the patient to establish a surgical pathway through the vessels to access surgical instruments such as catheters and guidewires. In many interventional procedures, patients require the use of anticoagulants or related medications to reduce the risks associated with blood coagulation, such as patients requiring atrial fibrillation ablation generally taking anticoagulant medications, and patients with coronary thrombi receiving thrombolytic drugs. These medications are used on the one hand for patient treatment and prevention of complications, and on the other hand, they cause difficulties in hemostasis at the puncture site. Generally, patients undergoing these procedures need to remain motionless for more than five hours after surgery to prevent complications such as subcutaneous hematoma and bleeding. However, prolonged immobility often causes significant inconvenience and discomfort to patients and may even pose a significant risk of other diseases, such as venous thrombosis, leading to more serious diseases (such as pulmonary embolism). Moreover, elderly patients constitute the majority of those requiring interventional procedures, and these difficulties and complications pose a greater risk and higher probability for the elderly. Therefore, hemostasis and closure of the vascular puncture site become crucial after interventional procedures.

Currently, there are devices on the market that first utilize an expandable or deployable mechanism to enter the vessel, temporarily seal the intra-vascular puncture site within the vessel, and then deliver expandable and degradable material into the tissue outside the vessel, utilizing physical expansion or chemical biological properties to achieve hemostasis. The structure used for temporary vascular occlusion is a nickel-titanium braided structure, which requires rear pulling control for deployment and occlusion.

However, the rear end of these products used to control deployment is a mechanical limit snap mechanism located at the distal end of the device. During use, the mechanism is pulled rearward to deploy the front temporary occlusion device, and the mechanism utilizes a weak limit snap structure to fix the relative distance pulled rearward. When retracting the temporary occlusion device, it is necessary to overcome the resistance of the limit snap to push the mechanism proximally. This mechanism has the following drawbacks: 1. Due to the gloves generally worn by operators during surgery being relatively smooth, the use of this mechanism requires forceful pinching to increase friction and then pulling it rearward, which is very inconvenient due to the need to overcome the resistance of the limit snap during the process. 2. Due to the weak limit snap mechanism not being able to completely limit its relative position, there is a certain probability that the mechanism automatically disengages from the snap, causing difficulties with the device during normal use. Furthermore, during operation, accidental contact with the mechanism may cause it to disengage from the snap, also leading to difficulties with use of the device.

Therefore, there is an urgent need to provide a new pulling control apparatus for a vascular closure device design that is safer, easier to use, and improves user experience during use of the device.

### SUMMARY

The purpose of the present device is to address the problem of difficult pulling operation and easy malfunction of the existing vascular closure device, and to provide a pulling control apparatus for a vascular closure device suitable for the pulling control of the front occlusion device of a vascular closure device, which can conveniently and stably perform the intended operation of the vascular closure device.

To achieve this purpose, the present invention provides a pulling control apparatus for a vascular closure device, comprising: a housing, a spring disposed within the housing for providing tensile force and tensile displacement, a combination of rotating components and pressing components for controlling different states, wherein the rotating components and pressing components have an axial displacement limitation after connection, and can rotate freely in the circumferential direction; a pulling wire can pass through the inside of the device and is fixed to the fixed component at the rear end of the pressing component; and by pressing the pressing component, the rotating component switches position, relying on the spring to provide tensile force, and transmits tensile force to the front end through the pulling wire.

Among the components, the housing is composed of a conical cap and a main body. The conical cap is a conical shell structure, and the rear end has a threaded hole to a through hole for fixing the pulling wire with screws. The catheter is connected to the conical cap. The conical shell structure is composed of a conical cover and a main body. The conical cover has a threaded structure at the rear end for connection with the main body, and a hole structure at the front end for assembly with a tube or catheter. The hole is characterized by a three-section structure, namely an expanding conical opening, the main body of the hole, and a rear narrowing conical opening. The expanding conical opening is located at the front end. Compared to the main body of the hole, it presents a conical expansion at an angle of 30° to 60°, with the length of the expanding component accounting for about one-third of the total length of the hole structure. The narrowing conical opening is located at the rear end of the hole structure. This narrowing conical opening is used for positioning to ensure accurate alignment during assembly with the tube or catheter. The center of the narrowing conical opening has a through hole through which the pulling wire can pass but which prevents the tube or catheter from passing through.

The front end of the shell main body has a threaded structure for connection with the conical cover. The interior rear end of the main body has multiple sets of grooves, with each set consisting of deep grooves and shallow grooves separated by intervals. The front end surface of the grooves is a beveled surface. The spring is in direct contact with the rotating component, and the preferred direction of the spring winding or helix is left-handed. The rotating component has guide rails distributed evenly around its circumference. The number of deep grooves is an integer multiple of the number of guide rails. The guide rails can only slide within the deep grooves, and their end surfaces are also beveled at the same angle as the front end surface of the grooves. The rear side of the guide rails has a serrated end surface, and the rear end of the rotating component has an elastic clip-like unidirectional connection structure. The front end of the actuating component, pressing component or pressing component assembly is a tubular structure with a serrated end surface, which meshes or mates with the serrated end surface of the rotating component. The rear side of the actuating component has a guide block, allowing the guide rails to slide within both the deep and shallow grooves. The rear end of the actuating component has an expanding conical mouth and a side slot structure. The fixing component is a cylindrical structure that matches the rear end of the actuating component, with a central through-hole and a threaded hole on the side leading to the through-hole, used for securing the traction wire with a set screw. The tube or catheter is connected to the conical cover.

The beneficial effect of the present invention is that the pulling control apparatus for a vascular closure device in the present application has more advantages compared to existing products. First, the present device is safer and easier to use. The force used for rearward pulling in the present device is provided by a spring within the main body. During operation, the operator only needs to press the rear end to perform expansion or contraction, avoiding the potential difficulty of use caused by insufficient friction due to the operator wearing gloves. In the present device, there is a very strong limiting mechanism, which will not cause automatic detachment from a mechanical principle. In the expanded state, the design of the main body has an automatic rebound effect, preventing temporary occlusion and contraction caused by misoperation. This avoids difficulties caused by some misoperations during the operation process.

Considering the above situation, the present device utilizes a clever mechanical structure to control the expansion of the front sealing device of the vascular closure device, and utilizes some structural designs to effectively ensure operation and potentially prevent functional operational difficulties. This improves the operability and safety of the vascular closure device.

In order to understand the features and advantages of the present device easier to understand, a preferred embodiment is specifically cited below, and a detailed description is made in conjunction with the attached drawings.

### DESCRIPTION OF THE DRAWINGS

In order to more clearly illustrate the specific embodiments of the present application or the technical solutions in the prior art, a simple introduction of the drawings used in the description of the specific embodiments or the prior art will be made below. It is obvious that the drawings described below are some embodiments of the present invention. For ordinary technicians in the field, other drawings can also be obtained based on these drawings without creative labor.
Fig. 1 is an overall schematic diagram of a product according to one embodiment of the present disclosure.
Fig. 2 is a schematic diagram of the appearance of an embodiment of the present disclosure.
Fig. 3 is a cross-sectional view of an embodiment of the present disclosure.
Fig. 4 is a cross-sectional view of the conical cap of an embodiment of the present disclosure.
Fig. 5 is a cross-sectional view of the main body of an embodiment of the present disclosure.
Figure 6 is a top view of a rotating component according to one embodiment of the present disclosure.
Figure 7 is a cross-sectional view of a pressing component according to one embodiment of the present disclosure.
Figure 8 is a schematic diagram of a fixing component according to one embodiment of the present disclosure.

### DETAILED DESCRIPTION

The following is a clear and complete description of the technical solution of the present device with reference to the accompanying drawings of the embodiments. It should be understood that the described embodiments are only a part of the embodiments of the present device, not all of them. The components of the present device shown and described in the accompanying drawings can be arranged and designed in various different configurations. Therefore, the detailed description of the selected embodiments provided in the accompanying drawings is not intended to limit the scope of the device as claimed. Any other embodiments obtained by those skilled in the art without making inventive steps, based on the described embodiments, are within the scope of protection of the present application.

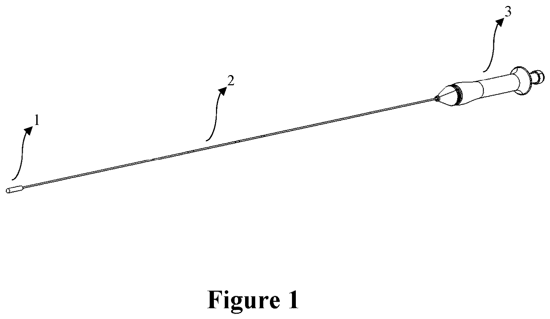

Firstly, please refer to Figure 1, which is an overall view of a product embodiment of the present device. In Figure 1, the front-end blocking structure 1 and the catheter 2 serve as supporting elements, while the pulling control apparatus for a vascular closure device 3 is used to control the expansion or contraction of the front-end blocking structure 1. The vascular closure device of the present invention features a front-end blocking structure 1 with a woven shape that can expand. The middle part is the catheter 2 that provides support. The front-end blocking structure 1 is characterized as a device that deforms under the tension of a pulling wire, which exerts a backward pulling force on the blocking structure 1. When the pulling wire is pulled, the blocking device expands to perform the blocking function, and when the pulling wire is relaxed and returns to its original position, the blocking structure 1 contracts. There are various possible structures for this blocking structure 1, which are not further described in the present application. Any device that meets the requirement of deforming and expanding under the tension of a pulling wire can be applied.

The pulling control apparatus for vascular closure device 3 of the present disclosure is a component used to control the vascular occluder to perform the expected expansion function. It provides tension and pulling distance to the pulling wire and is connected and fixed to the catheter 2, thereby controlling the expansion of the blocking structure 1.

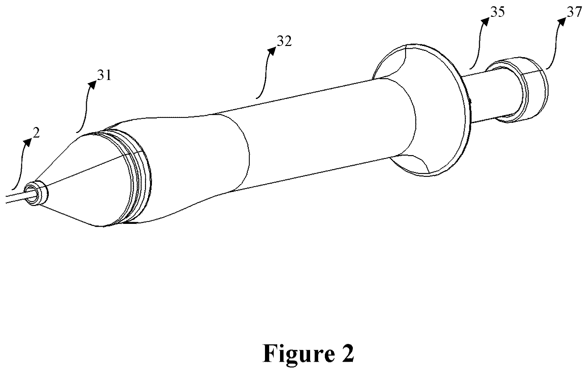

The pulling control apparatus for vascular closure device 3 mainly consists of a conical cap, a main body, a spring, a rotating component, an actuating or pressing component, a fixing component, and an end cap. Please refer to Figure 2, which is an appearance view of an embodiment of the present invention. The catheter 2 is connected to the conical cap 31, and the conical cap 31 is connected to the main body 32 by a threaded connection. The rear part includes the actuating component 35 and the end cap 37.

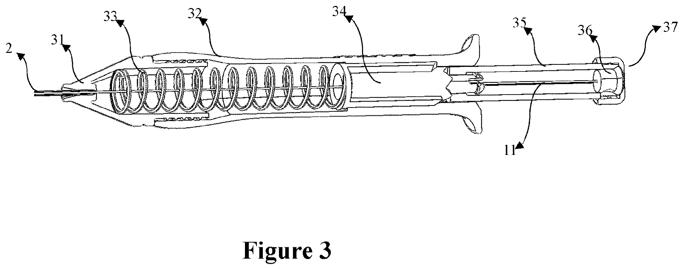

Figure 3 is a sectional view of an embodiment of the present device. The catheter 2 is connected to the conical cap 31, and the conical cap 31 is equipped with a spring 33 at the rear. The conical cap 31 is connected to the main body 32 by a threaded connection. The center of the main body 32 is a combination of the rotating component 34 and the actuating or pressing component 35. The fixing component 36 is embedded at the rear end of the actuating component 35. The pulling wire 11 is fixed to the fixing component 36 by a set screw, and the end cap 37 is connected to the actuating component 35 by a threaded connection.

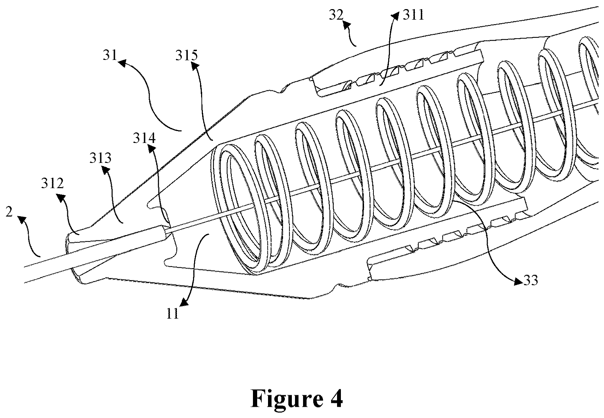

Figure 4 is a detailed sectional view of the conical cap of an embodiment of the present device. The catheter 2 is bonded and fixed to the front end of the conical cap 31. The hole structure of the conical cap 31 is mainly divided into three parts. The front end is an expanded conical opening 312, which presents a conical expansion of 30° to 60° relative to the main body of the hole structure. The length of the expanded part is about one-third of the total length of the hole structure. The middle part is the main body of the hole structure 313. The rear end is a conical opening 314, which is used for positioning to ensure accurate assembly of the catheter 2. The center of the conical opening 314 has a hole through which the pulling wire 11 can pass smoothly, but it prevents the catheter 2 from passing through. The rear end of the conical cap has a hollow structure 315 for assembling the spring 33, with a hole diameter slightly larger than the outer diameter of the spring 33. The conical cap has a conical front end and a cylindrical rear end with a threaded structure for connection to the main body. The conical cap is a shell-like structure with a hollow center. The front conical part has a hole structure for bonding connection with the catheter. To facilitate positioning and bonding with the catheter, the hole structure has the following special design. The front end of the hole structure is an expanded conical opening, which presents a conical expansion of 30° to 60° relative to the main body of the hole. This expanded structure is very conducive to the accumulation and curing of adhesive, ensuring a more reliable bond between the catheter and the conical cap. The middle part of the hole structure is the main body of the hole, with a diameter slightly larger (0.01 mm to 0.1 mm) than the diameter of the catheter to be assembled. This is a clearance fit, facilitating assembly and allowing some adhesive to seep in. The rear end is a conical opening for positioning to ensure accurate assembly of the catheter. The center of the conical opening has a hole through which the pulling wire can pass smoothly, but it prevents the catheter from passing through. The rear end of the conical cap has a hollow structure for assembling the spring, with a hole diameter slightly larger than the outer diameter of the spring.

The spring 33 provides the force and displacement required to expand the front-end blocking structure. Therefore, the design parameters of the spring should be based on the expansion force and relative displacement of the blocking structure. Since the spring needs to remain in a compressed state for a long time, to prevent stress relaxation from affecting the normal function of the device, the design parameters of the spring should minimize the internal strain of the spring when it is in a compressed state within the main body, that is, the effective number of turns of the spring should be increased as much as possible. The spring is in contact with the rotating component, so the winding direction of the spring should be consistent with the rotation direction of the rotating component, so that the edge of the spring does not block or interfere with the rotation of the rotating component.

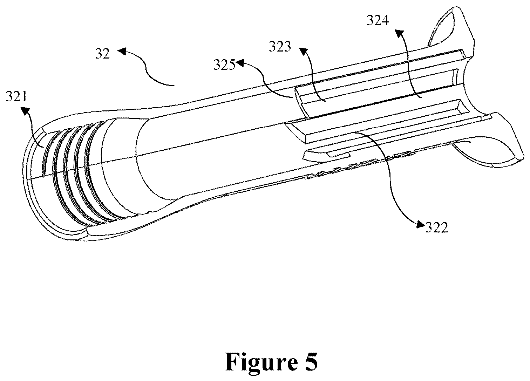

The main body, rotating component, and actuating or pressing component are the key components for action control. Figure 5 is a sectional view of the main body of an embodiment of the present device. The main body 32 is a hollow cylindrical shell with internal threads 321 at the front end for connection with the conical cap 31. The rear end has an even number of guide rail slots evenly distributed along the circumference. Each group of guide rail slots has the same parameters and consists of a deep slot 322 and a shallow slot 323, separated by an interval 324. The slots extend almost to the end of the main body but do not penetrate through it. The front end surface of each slot group is a sawtooth inclined plane 325, which is designed to match the inclined plane of the rotating component's guide rail end surface, with the same angle.

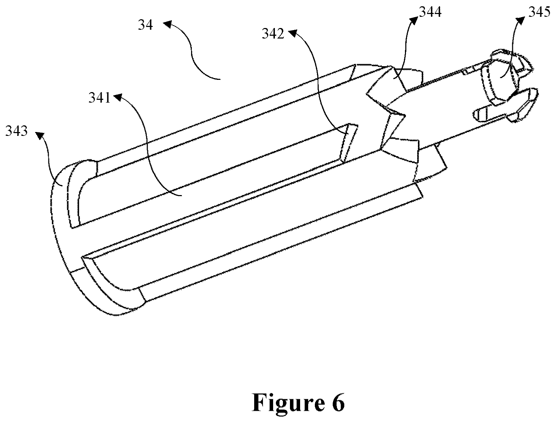

Figure 6 is a top view of the rotating component of an embodiment of the present device. The rotating component 34 is an overall hollow cylindrical component, divided into a guide rail section 341, a sawtooth or serrated end surface section 344, and a connection limit section 345. The guide rail section 341 is located at the front end of the component, with guide rails evenly distributed along the outer circumference of the cylinder. The guide rails match or mate within the deep slots of the main body, allowing relative sliding motion between the rotating component and the main body through the cooperation of the guide rails and slots. The number of deep slots is an integer multiple of the number of guide rails. At the front end of the rotating component, where the guide rails begin, there is a stepped structure 343 to limit the maximum sliding distance of the rotating component within the main body. The end surface 342 of the guide rail is an inclined plane that matches the end surface of the slot group. A certain distance away from the guide rail is the sawtooth end surface section 344, with sawteeth or serrations evenly distributed along the circumference. The number of sawteeth or serrations is the same as the total number of slots (deep slots plus shallow slots). The rear end of the rotating component is the connection limit section 345, which can be inserted into the inner hole of the actuating or pressing component. The connection limit section 345 has multiple sets of raised inclined step structures along the circumference. Using the elasticity of high molecular materials, these structures can be inserted into the stepped hole of the actuating component, entering and engaging with the stepped hole to form an assembly where the rotating component and the actuating component are connected. This connection allows axial displacement limitation but unrestricted rotation in the circumferential direction. The advantage of this mechanism is that the actuating component will not move axially, thus providing a good indication of the overall operating status.

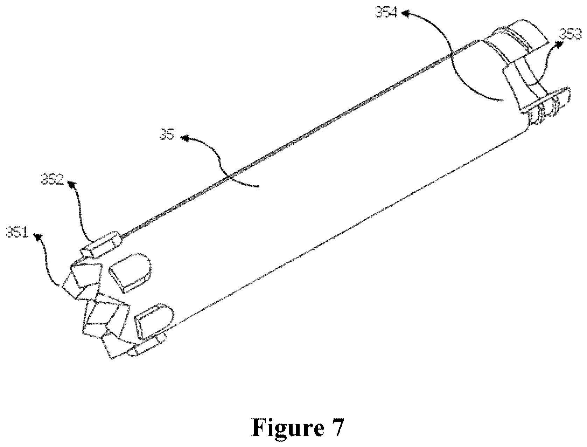

Figure 7 is a sectional view of a push-button component according to an embodiment of the present device. The push-button component 35 is an overall tubular structure, with a serrated end surface 351 at the front end. This serrated end surface 351 has the same dimensional parameters as the serrated end surface 354 of the rotating component, and they are designed to fit together. At a certain distance from the end surface, protruding sliding blocks 352 are uniformly distributed along the circumference. The number of sliding blocks 352 is consistent with the total number of all the grooves (including both deep and shallow grooves), and these sliding blocks 352 can fit into the shallow grooves 323, allowing the push-button component to slide relatively along the grooves. The centerline of the sliding block is offset from the centerline of the serrations by a certain angle. This structure enables the rotating component to rotate to the next position after the push-button component is pressed, thereby controlling the corresponding action. The interior of the push-button component has a stepped hole, which is designed to mate with the rotating component. The inner hole at the end of the push-button component has a conical flaring structure 353, which can be assembled with the fixing component 35. Additionally, the end has a slot-shaped opening for the installation of a set screw after assembly. The outer side of the end of the push-button component has threads, which are used for assembly with the end cap.

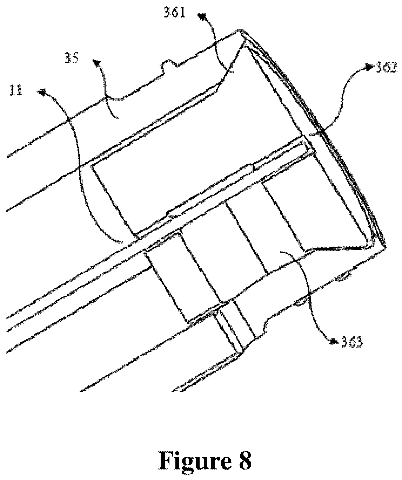

Figure 8 is a schematic diagram of the fixing part of an embodiment of the present invention. The fixing part is a conical cylindrical structure 361 at the end, with a through-hole 362 in the middle for the passage of the pulling wire 11, and a threaded hole 363 on the side for fixing the pulling wire with a set screw.

The end cap is a cap-shaped shell structure with internal threads for connection with the actuating component part.

The pulling control apparatus for vascular closure device has two control states. The normal state is to control the contraction of the front-end blocking structure of the vascular closure device. At this time, the spring 33 inside the main body 32 is compressed, and the guide rail inclined plane 342 of the rotating component 34 is located at the sawtooth inclined end surface 325 of the shallow slot 323. Since the guide rail 341 cannot slide along the shallow slot 323, the rotating component 34 is locked in the circumferential direction, and this state is stably maintained.

When it is necessary to expand the front-end mesh-like blocking structure of the vascular closure device, the actuating component 35 is pressed downward. The guide rail 341 of the rotating component 34 disengages from the sawtooth inclined end surface 325. Due to the certain offset angle between the centerline of the slider 352 of the actuating component 35 and the centerline of the sawtooth 351, the sawtooth end surface 351 of the rotating component 35 also has a certain offset with the sawtooth end surface 344 of the actuating component, causing the rotating component 34 to rotate slightly.

This causes the end surface 344 of the guide rail 341 to contact the inclined end surface 325 of the deep slot 322. The guide rail 341 of the rotating component 34 slides into the deep slot 322 along the inclined end surface 325. At this time, the spring 33 releases pressure, which is transmitted to the actuating component 35 and the fixing component 36, and acts on the pulling wire 11, causing the pulling wire 11 to provide a pulling force and displacement backward, thereby expanding the front-end mesh-like structure of the vascular closure device. The pulling control apparatus for vascular closure device is in the expansion control state. Pressing the actuating component 35 downward further to a certain position causes the guide rail 341 to disengage from the deep slot 322. Due to the offset of the sawtooth end surface 351, the rotating component rotates, causing the guide rail to enter the sawtooth inclined end surface 325 of the shallow slot 323, and the rotating component 34 is locked, returning to the contraction control state.

Although the embodiments of the present invention have been disclosed as described above, they are not limited to the applications listed in the specification and embodiments. It can be fully applied to various fields suitable for the present invention. Additional modifications can be easily realized by those skilled in the field. Therefore, the present invention is not limited to the specific details and the embodiments shown and described here, as long as it does not depart from the general concept defined by the claims and their equivalents.

## Claims

1. A pulling control apparatus for vascular closure device, comprising:
a housing, a spring disposed within the housing for providing tensile force and tensile displacement,
a rotating component and a pressing component assembly for controlling different states, wherein the rotating component and pressing component assembly are axially limited after connection and can rotate freely in the circumferential direction; a pulling wire can pass through the interior of the device and is fixed to a fixed component at a rear end of the pressing component assembly; and
wherein upon pressing the pressing component assembly, the rotating component switches positions, relying on the spring to provide tensile force and transmitting the tensile force to a front end of the pressing component assembly through the pulling wire.

2. The pulling control apparatus for vascular closure device as claimed in claim 1, wherein the housing comprises a conical cap and a main body, the conical cap is a conical shell structure, having a rear end with a threaded structure connected to the main body, and a front end having a hole structure enabling assembling with a catheter assembly, and the hole structure is a three-segment structure, namely an expanded conical opening, a hole main body, and a rear contracted conical opening.

3. The pulling control apparatus for vascular closure device as claimed in claim 2, further comprising an expanded conical opening located at a very front end of the hole structure, and the expanded conical opening enables a conical expansion in the expanded conical opening of 30° to 60° relative to the hole main body, and the length of the expanded conical opening accounts for about one-third of the length of the hole structure.

4. The pulling control apparatus for vascular closure device as claimed in claim 2, wherein the rear contracted conical opening is located at a rear end of the hole structure, and the rear contracted conical opening enables positioning, so that the catheter assembly can be accurately positioned during assembly; and
wherein a hole is left in the center of the rear contracted conical opening through which the pulling wire can pass, and the hole allows the pulling wire to pass but can prevent the catheter from passing.

5. The pulling control apparatus for vascular closure device as claimed in claim 1, wherein a front end of an outer shell body is connected to a conical cap via a threaded structure, and an internal rear end has multiple sets of slots, each set of slots divided into deep slots and shallow slots, separated by partitions in a middle section of the internal rear end; and wherein a surface of the front end is a slope.

6. The pulling control apparatus for vascular closure device as claimed in claim 1,wherein the spring is in direct contact with the rotating component, and a helix direction of the spring is preferably left-handed.

7. The pulling control apparatus for vascular closure device as claimed in claim 5, wherein the rotating component is uniformly provided and distributed with rails in the circumferential direction, and the number of deep slots is an integer multiple of the number of rails, the rails can only slide within the deep slots, and a rail end surface is a slope, having an angle of the same slope as the slope of the surface of the front end of the slot; and a rear side of the rail has a serrated end surface, and a rear end of the rotating component has a resilient snap-fit one-way connecting structure.

8. The pulling control apparatus for vascular closure device as claimed in claim 7, wherein the front end of the pressing component assembly is a tubular structure with a serrated end surface, which pressing component assembly serrated end surface cooperates with the serrated end surface of the rotating component; the rear end of the pressing component assembly has a guide block, and a rail can slide within the deep slots and shallow slots; and the rear end of the pressing component assembly has a flared conical opening and a side slot structure.

9. The pulling control apparatus for vascular closure device as claimed in claim 1, wherein the fixed component at the rear end of the pressing component is a cylindrical structure matching the rear end of the pressing component, with a through hole in a center and threaded holes on a side leading to the through hole, for securing a fixing screw to the pulling wire.

10. The pulling control apparatus for vascular closure device as claimed in claim 2, wherein a catheter is connected to the conical cap.
